# EUROPEAN PATENT APPLICATION

(11) **EP 4 501 340 A1**
(43) Date of publication of application: **05.02.2025**
(21) Application number: 23780127.9
(22) Date of filing: 24.03.2023
(51) Int. Cl.: A61K 36/185, A61K 8/49, A61K 8/9789, A61K 31/37, A61P 3/04, A61P 17/00, A61P 29/00, A61P 35/00, A61P 39/06

(54) **METHOD FOR PRODUCING ELLAGIC ACID-CONTAINING COMPOSITION AND ELLAGIC ACID-CONTAINING COMPOSITION**

(30) Priority: 28.03.2022 JP 2022051408
(71) Applicant: Sumitomo Chemical Company, Limited, Tokyo 103-6020 (JP)
(72) Inventor: YONEYAMA, Toshihiro, Osaka-shi, Osaka 554-8558 (JP); KUSUMOTO, Masanori, Osaka-shi, Osaka 554-8558 (JP); AOKI, Mikio, Osaka-shi, Osaka 554-8558 (JP); MIKATA, Kazuki, Osaka-shi, Osaka 554-8558 (JP)
(74) Representative: Hoffmann Eitle
(86) International application number: PCT/JP2023/011738
(87) International publication number: WO 2023/190117

(57) **Abstract**

An object of the present disclosure is to provide a novel method for producing an ellagic acid-containing composition, the method capable of improving the solubility of ellagic acid. The present embodiment is a method for producing an ellagic acid-containing composition, including: a step of providing a solution containing at least an aqueous medium and a raw material derived from a plant of the genus *Terminalia,* containing ellagic acid; and a step of heat-treating the solution. The present embodiment is the production method, further including a step of a hydrolysis treatment to hydrolyze ellagitannin contained in the raw material to produce additional ellagic acid, wherein the hydrolysis-treated solution is heat-treated.

## Description

### Technical Field

The present disclosure relates to a method for producing an ellagic acid-containing composition and an ellagic acid-containing composition.

### Background Art

Ellagic acid is a promising natural compound that has been reported to have various effects, including anti-cancer, anti-inflammatory, antioxidant, anti-obesity, and skin-whitening effects.

For example, Patent Literature 1 discloses that ellagic acid has excellent physiological activities, such as antioxidant activity against edible oils and fats, antimutagenic activity against microorganisms, and antitumor activity against small animals such as mice and rats, and is a useful compound in the food and pharmaceutical industries.

However, ellagic acid has low solubility in water, making it difficult to use in water-based products. Therefore, studies are being performed to improve the solubility of ellagic acid.

For example, Patent Literature 2 discloses a method for producing an ellagic acid composition, the method including: a step of mixing a raw material containing a guava leaf extract and containing 1 to 5% by mass of free ellagic acid in the solid content with an aqueous medium to prepare a raw material for heat treatment; and a step of heat-treating the raw material for heat treatment at 100 to 180°C.

### Citation List

### Patent Literature

Patent Literature 1: Japanese Patent Laid-Open No. 05-331183
Patent Literature 2: Japanese Patent Laid-Open No. 2015-033375

### Summary of Invention

### Problems to be Solved by Invention

However, the method of Patent Literature 2 is limited to raw materials that contain a guava leaf extract and 1 to 5% by mass of free ellagic acid in the solid content. In addition, the method of Patent Literature 2 requires heating at a high temperature of 100 to 180°C. Therefore, there is a demand for a novel method for producing an ellagic acid-containing composition, the method capable of improving the solubility of ellagic acid.

Therefore, the present disclosure has been made in consideration of at least one of the above problems, and an object of the present disclosure is to provide a novel method for producing an ellagic acid-containing composition, the method improving the solubility of ellagic acid.

### Means to Solve the Problems

Examples of aspects of the present embodiment are as follows.
(1) A method for producing an ellagic acid-containing composition, comprising: a step of providing a solution containing at least an aqueous medium and a raw material derived from a plant of the genus *Terminalia,* containing ellagic acid; and a step of heat-treating the solution.
(2) The production method according to (1), further comprising a step of a hydrolysis treatment to hydrolyze ellagitannin contained in the raw material to produce additional ellagic acid, wherein the hydrolysis-treated solution is heat-treated.
(3) The production method according to (2), wherein the hydrolysis treatment is hydrolysis with an acid.
(4) The production method according to any one of (1) to (3), wherein the solution further contains ellagic acid added separately from the ellagic acid contained in a raw material derived from a plant of the genus *Terminalia.*
(5) The production method according to any one of (1) to (4), wherein the plant of the genus *Terminalia* is *Terminalia catappa.*
(6) The production method according to any one of (1) to (5), wherein the raw material derived from a plant of the genus *Terminalia* is at least one material selected from leaves, fruits, stems, and branches of a plant of the genus *Terminalia,* or is an extract extracted from at least one material selected from leaves, fruits, stems, and branches of a plant of the genus *Terminalia* with an extraction medium.
(7) The production method according to any one of (1) to (6), wherein the raw material derived from a plant of the genus *Terminalia* is an extract extracted from at least one material selected from leaves, fruits, stems, and branches of a plant of the genus *Terminalia* with an extraction medium.
(8) The production method according to (6) or (7), wherein the extraction medium is water, an aqueous medium, or a mixed solvent thereof.
(9) The production method according to any one of (6) to (8), wherein the extraction medium is water, an alcohol, or a mixed solvent of water and an alcohol.
(10) The production method according to any one of (1) to (9), wherein a temperature for the heat treatment is 60°C or more.
(11) The production method according to any one of (1) to (10), wherein a temperature for the heat treatment is less than 100°C.
(12) The production method according to any one of (1) to (11), wherein the aqueous medium in the solution is water, alcohol, or a mixed solvent of water and alcohol.
(13) The production method according to any one of (1) to (12), further comprising a step of concentration-treating a solution after the heat-treating step.
(14) The production method according to any one of (1) to (13), wherein a concentration of ellagic acid dissolved in a solution obtained by the heat-treating step, or the concentration-treating step when performing the concentration treatment is 50 µg/mL or more.
(15) The production method according to any one of (1) to (14), further comprising a step of dry-treating the solution, after the heat-treating step, or after the concentration-treating step when performing the concentration treatment, to obtain an ellagic acid-containing solid.
(16) An ellagic acid-containing composition obtained by the production method according to any one of (1) to (15).
(17) A medicine, cosmetic, quasi-drug, or food or drink comprising the ellagic acid-containing composition according to (16).
(18) Use of a raw material derived from a plant of the genus *Terminalia* for solubilizing ellagic acid, wherein a solution containing a raw material derived from a plant of the genus *Terminalia* and an aqueous medium is heat-treated in the presence of ellagic acid.

### Effects of Invention

The present disclosure can provide a novel method for producing an ellagic acid-containing composition, the method capable of improving the solubility of ellagic acid.

### Embodiments for Carrying out Invention

The present embodiment is a method for producing an ellagic acid-containing composition, comprising: a step of providing a solution containing at least an aqueous medium and a raw material derived from a plant of the genus *Terminalia,* containing ellagic acid; and a step of heat-treating the solution.

The present embodiment can provide a novel method for producing an ellagic acid-containing composition, the method capable of improving the solubility of ellagic acid. The present inventors have found that the effect of solubilizing ellagic acid can be obtained by heat-treating a solution containing a raw material derived from a plant of the genus *Terminalia* and an aqueous medium in the presence of ellagic acid, and reached the present embodiment. The reason why the effect of solubilizing ellagic acid can be obtained by heat-treating a solution containing raw materials derived from a plant of the genus *Terminalia* and ellagic acid is assumed to be that the components contained in the raw materials act on ellagic acid by the heat treatment, solubilizing the ellagic acid. It is assumed that the components contained in the raw materials act on ellagic acid through the formation of inclusion complexes, the formation of salts, the formation of associations, or the like. This assumption does not limit the present embodiment.

The production method according to the present embodiment will be described in detail below.

The present embodiment includes a step of providing a solution containing at least an aqueous medium and a raw material derived from a plant of the genus *Terminalia,* containing ellagic acid.

The form of providing the solution is not particularly limited, and includes, for example, a form in which the solution is provided by mixing at least a raw material derived from a plant of the genus *Terminalia* and an aqueous medium, or a form in which the solution is provided by purchasing the solution.

A plant of the genus *Terminalia* is a tree that contains a high content of ellagic acid. In the present embodiment, a plant of the genus *Terminalia* is preferably *Terminalia catappa,* because of containing a high content of ellagic acid.

In the present description, ellagic acid is used to include free ellagic acid, the salt thereof, and the solvate thereof (including hydrate). Many ellagitannins, which have a structure in which ellagic acid is bound to sugar, are present in the plant of the genus *Terminalia,* but in the preset description, ellagic acid and ellagitannin are used separately.

The raw material derived from a plant of the genus *Terminalia* is not particularly limited, and examples thereof include at least one material selected from the leaves, fruits, stems, and branches of a plant of the genus *Terminalia,* and an extract extracted from at least one material selected from the leaves, fruits, stems, and branches of a plant of the genus *Terminalia* with an extraction medium. As at least one material selected from the leaves, fruits, stems, and branches, for example, cut products, crushed products, ground products, crushed products, and the like can be used. The material may be used singly or may be used in combination of two or more types.

An extract derived from a plant of the genus *Terminalia* can be prepared by extracting the contained components from at least one material selected from leaves, fruits, stems, and branches with an extraction medium (e.g., an aqueous medium such as water or ethanol). As the raw material derived from a plant of the genus *Terminalia,* it is preferable to use an extract, and in particular, an extract extracted using water, an aqueous medium, or a mixed solvent thereof as the extraction medium is preferable. An extract derived from a plant of the genus *Terminalia* contains a high content of ellagic acid derived from a plant of the genus *Terminalia.*

The extract derived from a plant of the genus *Terminalia* is preferably an extract of *Terminalia catappa* leaves. *Terminalia catappa* leaves are a material containing a high content of ellagic acid derived from *Terminalia catappa.* The *Terminalia catappa* leaf extract is preferably one extracted using water, an aqueous medium, or a mixed solvent thereof as the extraction medium.

The extraction method is not particularly limited, and examples thereof include ultrasonic extraction, microwave extraction, solid-liquid extraction, liquid-liquid extraction, immersion extraction, decoction extraction, percolation extraction, steam distillation extraction, reflux extraction, and stirring extraction. In addition, the extraction method may be heated extraction, room temperature extraction, or cold extraction. The extraction temperature is not particularly limited, but is preferably 0°C or more, and more preferably 20°C or more, in terms of increasing the extraction efficiency. In addition, the extraction temperature is, for example, 100°C or less.

The extraction medium is not particularly limited, and examples thereof include aqueous media such as water (including water vapor) or alcohol, subcritical or supercritical carbon dioxide, and edible oils and fats such as soybean oil, rapeseed oil, sunflower oil, palm oil, and lard. Among them, it is preferable to use water, an aqueous medium, or a mixed solvent of these as the extraction medium. Examples of the alcohol as the aqueous medium include ethanol and propanol. For example, an aqueous ethanol solution of 30 to 70 v/v% can also be used as the extraction medium. In addition, a solvent with the pH adjusted by adding an acid or alkali to the above extraction solvent may be used. The extraction medium may be used singly or in combination of two or more types.

The extract may be used as it is in the form of an extracted solution, or may be subjected to treatment such as purification, concentrating (vacuum concentrating, membrane concentrating, or the like), dilution, or filtration, as necessary. Further, the extracted solution may be subjected to treatment such as spray drying, freeze drying, or concentrating to dryness, and used as a dried product. For example, the extract may be prepared as an extract liquid containing the extraction medium, or as a solid from which the extraction medium has been removed (e.g., a freeze-dried product or a spray-dried product).

The content of ellagic acid in the extract is preferably 2% or more, preferably 5% or more, and preferably 10% or more, on a basis of dry weight. In addition, the content of ellagic acid in the extract is preferably 25% or less, preferably 20% or less, and preferably 15% or less, on a basis of dry weight.

The aqueous medium is water, an organic solvent that mixes uniformly with water, or a mixed solvent of these. Examples of water include tap water, distilled water, ion-exchanged water, and purified water. The water may be one that contains solutes such as salts, sugars, and pH adjusters. The organic solvent is not particularly limited as long as it mixes uniformly with water, and examples thereof include alcohol. Examples of the alcohol include monohydric alcohols such as ethanol or propanol, dihydric alcohols such as propylene glycol or butylene glycol, and trihydric alcohols such as glycerin. For example, an aqueous solution of 30 to 70 v/v% ethanol can be used as the aqueous medium. The aqueous medium may be used singly, or may be used in combination of two or more types.

One aspect of the present embodiment may further include a step of a hydrolysis treatment to hydrolyze ellagitannins contained in the raw material to produce further ellagic acid. In this case, the hydrolysis-treated solution is heat-treated as described below. Hydrolyzing ellagitannins contained in the raw material can further increase the concentration of ellagic acid in the solution.

The hydrolysis treatment is not particularly limited as long as it can hydrolyze ellagitannins contained in the raw material to produce further ellagic acid, but examples of the hydrolysis treatment include hydrolysis with an acid and hydrolysis with an enzyme. The hydrolysis may be further promoted by a heat treatment described later.

For the hydrolysis with an acid, the acid is one added to hydrolyze ellagitannin contained in the raw material to produce ellagic acid. In addition, the hydrolysis with the acid may be further promoted by a heat treatment described later.

The acid is not particularly limited, but examples thereof include hydrochloric acid and sulfuric acid. The acid may be used singly or may be used in combination of two or more types.

In the case of hydrolysis with an acid, the pH of the solution (20°C) is not particularly limited as long as the hydrolysis reaction occurs, but is, for example, 5 or less. From the viewpoint of efficiently causing the hydrolysis reaction of ellagitannin, the pH of the solution (20°C) is preferably 4 or less, more preferably 3 or less, more preferably 2 or less, and more preferably 1 or less.

For hydrolysis by an enzyme, the enzyme used for hydrolysis is not particularly limited as long as it can hydrolyze ellagitannin to produce ellagic acid, and examples thereof include tannase. Tannase can be isolated or prepared, for example, from a fungal or bacterial source. Examples of tannase include tannase obtained by culturing tannase-producing fungi of the genera *Aspergillus, Penicillium,* and *Rhizopus.* Among them, those derived from *Aspergillus oryzae* are preferable. The concentration of tannase in the solution is not particularly limited, but is, for example, 10 units/L to 500 units/L, and preferably 20 units/L to 150 units/L.

In one aspect of the present embodiment, the solution may further contain ellagic acid added separately from the ellagic acid contained in the raw material derived from a plant of the genus *Terminalia.* One aspect of the present embodiment may include a step of further adding ellagic acid to the solution separately from the ellagic acid contained in the raw material, thereby allowing to obtain an ellagic acid-containing composition having a higher ellagic acid concentration.

For the amount of ellagic acid added in the solution, from the viewpoint of increasing the concentration of ellagic acid in the resulting ellagic acid-containing composition, it is preferable to add ellagic acid such that the content of ellagic acid in the solution after the addition is preferably 5% by mass or more, preferably 6% by mass or more, preferably 7% by mass or more, preferably 8% by mass or more, preferably 9% by mass or more, and preferably 10% by mass or more, on a basis of the solid content. For the amount of ellagic acid added in the solution, it is preferable to add ellagic acid such that the content of ellagic acid in the solution after the addition is preferably 20% by mass or less, preferably 19% by mass or less, preferably 18% by mass or less, preferably 17% by mass or less, preferably 16% by mass or less, and preferably 15% by mass or less, on a basis of the solid content.

Ellagic acid may be added by adding a compound made of ellagic acid itself (including free ellagic acid, the salt thereof, and the solvate thereof), or by adding a material containing ellagic acid (e.g., an extract from a certain plant). For example, ellagic acid is commercially available from Fujifilm Wako Pure Chemical Industries, Ltd., and ellagic acid dihydrate is commercially available from Tokyo Chemical Industry Co., Ltd. In addition, for example, a material containing ellagic acid is commercially available from Sabinsa Japan Corporation as pomegranate ellagic acid.

The aspect of performing the hydrolysis treatment and the aspect of further adding ellagic acid may be appropriately combined. Therefore, the resulting ellagic acid-containing composition can contain ellagic acid contained in the raw material, ellagic acid produced by the hydrolysis reaction of ellagitannin, and ellagic acid derived from ellagic acid added separately from the ellagic acid contained in the raw material derived from a plant of the genus *Terminalia,* and can have a higher ellagic acid concentration.

The content of the raw material (particularly the extract) derived from a plant of the genus *Terminalia* in the solution is preferably 60% by mass or more, preferably 70% by mass or more, and preferably 80% by mass or more, on a basis of the solid content, from the viewpoint of improving the solubility of ellagic acid. The content of the raw material (particularly the extract) derived from a plant of the genus *Terminalia* in the solution is preferably 100% by mass or less, preferably less than 100% by mass, and preferably 99% by mass or less. In the present description, the solid content of the raw material derived from a plant of the genus *Terminalia* refers to the dry mass in a case where the raw material derived from a plant of the genus *Terminalia* is in a solid form, and refers to the mass after drying the raw material derived from a plant of the genus *Terminalia* in an electric thermostatic dryer at 105°C for 3 hours to remove volatile components in a case where the raw material derived from a plant of the genus *Terminalia* is in a form other than a solid (e.g., liquid or paste form).

The total content of the solid in the solution is not particularly limited, but is, for example, 0.5 g/L or more and 50 g/L or less.

The solution may contain other components in addition to the above components. The other components may be added to an extent that does not impair the effect of the present embodiment, and the content of the other components in the solution is, for example, 20% by mass or less, preferably 15% by mass or less, preferably 10% by mass or less, preferably 5% by mass or less, and preferably 1% by mass or less. Examples of the other component include an organic solvent in the ellagic acid-containing liquid used to contain a predetermined amount of ellagic acid in the solution.

The present embodiment includes a step of heat-treating the solution.

As described above, the heat treatment has an effect of solubilizing ellagic acid. The reason for this is assumed to be that the components contained in the raw material act on ellagic acid through the formation of an inclusion complex, the formation of a salt, the formation of association, or the like by the heat treatment, thereby solubilizing the ellagic acid. This assumption does not limit the present embodiment.

The temperature for the heat treatment is preferably 60°C or more, preferably 65°C or more, preferably 70°C or more, preferably 75°C or more, and preferably 80°C or more, from the viewpoint of improving the solubility of ellagic acid. The temperature for the heat treatment is preferably 180°C or less, preferably 170°C or less, and preferably 160°C or less, from the viewpoint of the thermal stability of the component. Further, the temperature for the heat treatment is preferably less than 100°C, preferably 99°C or less, and preferably 98°C or less, mainly from the viewpoint of energy costs. The present embodiment is particularly excellent in that an excellent ellagic acid solubilization effect can be obtained even if the temperature for the heat treatment temperature is less than 100°C. The time for the heat treatment may vary depending on the treatment method and temperature, but is, for example, 10 minutes to 10 hours. These temperature and time conditions are merely examples, and can be appropriately set in consideration of the relationship between temperature and time and other factors. The enzyme used in the hydrolysis treatment can also be inactivated by the heat treatment.

The heat treatment method is not particularly limited, and known methods can be applied. In addition, heating can be performed under normal pressure or under increased pressure. In addition, heat treatment can be performed by steam heating, and a continuous or batch type steamer or autoclave can be used.

The heat-treated solution may be cooled appropriately. The solution temperature after cooling is preferably 50°C or less, and more preferably 30°C or less. The cooling method is not particularly limited, and may be, for example, cooling with a cooler or leaving at room temperature.

The concentration of ellagic acid in the solution obtained after the heat treatment is preferably 50 µg/mL or more, more preferably 60 µg/mL or more, more preferably 70 µg/mL or more, more preferably 80 µg/mL or more, more preferably 90 µg/mL or more, and more preferably 100 µg/mL or more.

As described above, the step of providing the solution, the hydrolysis-treating step, and the heat-treating step are at least shown as the steps of the present embodiment, but the order of these steps is not particularly limited as long as the effect of the present embodiment is not impaired. For example, in the present embodiment, it is basically assumed that the step of providing the solution, the hydrolysis-treating step, and the heat-treating step are performed separately, but it is not essential that these are performed separately in that order. For example, the present embodiment may also include a case where the step of providing the solution and the heat-treating step are performed simultaneously. As a specific example, a raw material derived from a plant of the genus *Terminalia* may be added while the aqueous medium is heated. In addition, for example, the present embodiment may include a case where the step of providing the solution and the hydrolysis-treating step are performed simultaneously. As a specific example, a raw material derived from a plant of the genus *Terminalia* may be added to a liquid containing an aqueous medium and an acid or an enzyme. In addition, for example, the present embodiment may include a case where the step of a hydrolysis treatment and the step of a heat treatment are performed simultaneously. As a specific example, an acid or an enzyme may be added while the solution is heated. In addition, for example, the present embodiment may include a case where the step of providing the solution, the hydrolysis-treating step, and the heat-treating step are performed simultaneously. As a specific example, a raw material derived from a plant of the genus *Terminalia* may be added while heating an aqueous medium, an acid, or a liquid containing an enzyme. Further, as described above, a form in which the solution may further contain ellagic acid added separately from the ellagic acid contained in the raw material derived from a plant of the genus *Terminalia* has been shown, but the separately added ellagic acid may be contained in the solution in the step of providing the solution, in the solution in the hydrolysis-treating step, or in the solution in the heat-treating step, and is not particularly limited as long as the ellagic acid added separately during the heat treatment is present in the solution.

The obtained solution is subjected to purification treatment, concentration treatment, drying treatment, or the like as necessary to obtain an ellagic acid-containing composition.

In the present embodiment, the purification treatment may include a step of removing an undissolved remained solid portion from the heat-treated solution. The removal method is not particularly limited, but examples thereof include centrifugation, decantation, and filtration.

In the present embodiment, the concentrating treatment may include a step of concentration-treating the above heat-treated solution. The ellagic acid-containing composition may be in the form of a solution or in the form of a paste. Examples of the concentrating method of the solution include general methods such as vacuum concentrating (e.g., vacuum centrifugation) and membrane concentrating.

The concentration of ellagic acid in the ellagic acid-containing composition after the concentration treatment may be, for example, 500 µg/mL or more. The concentration of ellagic acid in the ellagic acid-containing composition after the concentrating treatment is preferably 750 µg/mL or more, preferably 800 µg/mL or more, preferably 850 µg/mL or more, preferably 900 µg/mL or more, and preferably 950 µg/mL or more. Due to the solubilizing effect of ellagic acid, the ellagic acid-containing composition obtained in the present embodiment does not precipitate ellagic acid even after concentration, and ellagic acid can be present in a dissolved state.

In the present embodiment, the drying treatment may include a step of dry-treating the solution to obtain an ellagic acid-containing solid. The ellagic acid-containing composition can be in the form of a solid such as a powder or granules. Examples of the method for removing the medium include freeze-drying, evaporation to dryness, and spray drying. The drying step may be performed after the concentrating treatment. The obtained solid may be classified, granulated, or pulverized or the like, as necessary.

The ellagic acid-containing composition according to the present embodiment obtained as a solid has excellent solubility of ellagic acid in water even when added to water again. This indicates that even in a case where the ellagic acid-containing composition according to the present embodiment is made into a solid, ellagic acid is present in the solid in a form that can be easily solubilized.

One aspect of the present embodiment can be an ellagic acid-containing composition obtained by the production method according to the present embodiment. Another aspect of the present embodiment can be a drug, cosmetic, quasi-drug, or food or drink containing the ellagic acid-containing composition. In the present embodiment, the term "food or drink" refers to not only general food or drink, but also a food other than a drug and quasi-drug that can be taken for the purpose of maintaining or improving health (e.g., health foods, functional foods, health-promoting foods, or foods for special uses). Health foods include foods provided under names such as nutritional supplements, dietary supplements, and supplements. Health-promoting foods are defined by the Food Sanitation Act or the Food Promotion Act, and include foods for specified health uses and foods with nutritional functions that can display specific health effects, functions of nutritional components, reduction of disease risk, and the like. The form of the food or drink may be any form suitable for consumption, such as solid, liquid, granular, powder, capsule, cream, or paste.

### Examples

The present embodiment will be described below using examples. The specific aspects of the present embodiment are not limited to those of examples, and the configuration may be modified as appropriate within the scope of the spirit of the present disclosure.

### [Measurement method]

### (Ellagic acid concentration)

Quantification of free ellagic acid was performed using a liquid chromatograph (manufactured by Thermo Fisher Scientific Inc.) equipped with a ACQUITY^{™} PREMIER HSS T3 (2.1 × 100 mm) column (manufactured by Waters Corporation) at a column temperature of 40°C by gradient method. Mobile phase solution A was 0.05% acetic acid aqueous solution, and solution B was acetonitrile containing 0.05% acetic acid, delivered at 250 µL/min. Gradient conditions were as follows.

**[Table 1]**

| Time (minutes) | Solution A (%) | Solution B (%) |
|---|---|---|
| 0 | 98 | 2 |
| 20 | 40 | 60 |
| 28 | 1 | 99 |
| 30 | 1 | 99 |
| 31 | 98 | 2 |
| 37 | 98 | 2 |

A sample injection volume was set to 2 µL, and quantification was performed on a basis of absorbance at a wavelength of 252 nm.

### (pH)

The pH was measured using a pH meter (SPH70, manufactured by Asone International Inc.) after the sample temperature was adjusted to 20°C.

### (Preparation Example 1: 50% ethanol extract)

As a plant of the genus *Terminalia,* crushed *Terminalia catappa* leaves (collected from Henza Island (Yonashiro Henza, Uruma City, Okinawa Prefecture)) were used. A dispersion was prepared by adding 5 mL of 50% ethanol aqueous solution to 100 mg of crushed *Terminalia catappa* leaves. The dispersion was then subjected to ultrasonic treatment for 1 hour. Then, the dispersion after ultrasonic treatment was filtered to obtain an extract liquid. The extract liquid was then concentration-treated using a vacuum centrifuge, and then subjected to freeze-drying treatment to obtain a freeze-dried product. The freeze-dried product was used as an extract derived from a plant of the genus Terminalia. In a case of the use in the following experiments, the freeze-dried product was dissolved and dispersed in a 50% ethanol aqueous solution so as to have a concentration of 10 mg/mL to prepare an extract-containing liquid (10 mg/mL).

### (Preparation Example 2: Water extract)

As a plant of the genus Terminalia, crushed leaves of *Terminalia catappa* (collected from Henza Island (Yonashiro Henza, Uruma City, Okinawa Prefecture)) were used. A dispersion was prepared by adding 5 mL of pure water to 100 mg of crushed *Terminalia catappa* leaves. The dispersion was then subjected to ultrasonic treatment for 1 hour. Then, the dispersion after ultrasonic treatment was filtered to obtain an extract liquid. The extract liquid was then concentration-treated using a vacuum centrifuge, and then subjected to freeze-drying treatment to obtain a freeze-dried product. The freeze-dried product was used as an extract derived from a plant of the genus Terminalia. In a case of the use in the following experiments, the freeze-dried product was dissolved and dispersed in pure water so as to have a concentration of 10 mg/mL to prepare an extract-containing liquid (10 mg/mL).

### (Preparation Example 3: 100% ethanol extract)

As a plant of the genus Terminalia, crushed leaves of *Terminalia catappa* (collected from Henza Island (Yonashiro Henza, Uruma City, Okinawa Prefecture)) were used. A dispersion was prepared by adding 5 mL of 100% ethanol to 100 mg of crushed *Terminalia catappa* leaves. Then, the dispersion was subjected to ultrasonic treatment for 1 hour. Then, the dispersion after ultrasonic treatment was then filtered to obtain an extract liquid. Then, the extract liquid was concentration-treated using a reduced pressure centrifuge and then subjected to freeze-drying treatment to obtain a freeze-dried product. The freeze-dried product was used as an extract derived from a plant of the genus Terminalia. In a case of the use in the experiments described below, the freeze-dried product was dissolved and dispersed in a 100% aqueous ethanol solution so as to have a concentration of 10 mg/mL to prepare an extract-containing liquid (10 mg/mL).

### (Preparation Example 4: Ellagic acid-containing Liquid)

Ellagic acid was used from Fujifilm Wako Pure Chemical Industries, Ltd. (Cat. No. 057-08751). In a case of the use in the experiment, ellagic acid was dissolved in pyridine so as to have a concentration of 1 mg/mL to prepare an ellagic acid-containing liquid (1 mg/mL).

### [Investigation of increase in ellagic acid concentration by hydrolysis treatment]

### (Example 1)

A solution was prepared in a 1.5 mL tube by mixing 50 µL (corresponding to 500 µg of extract) of the extract-containing liquid (10 mg/mL, containing 50% ethanol extract) prepared in Preparation Example 1 with 50 µL of pyridine. The solution in the tube was subjected to reduced pressure centrifugation treatment to remove the solvent to prepare an extract-containing powder. Then, 500 µL of hydrochloric acid (2 N) was added to the tube and mixed with the extract-containing powder to prepare a mixed solution. Then, the tube containing the mixed solution was placed in a block incubator and incubated at 95°C for 4 hours, thereby performing a hydrolysis treatment. Then, the mixed solution after incubation was left in the ambient environment to return to room temperature (23°C). Thereafter, the mixed solution was filtered through a 0.45 µm filter to remove solids, and a liquid composition E1 was obtained.

### (Comparative Example 1)

A solution was prepared in a 1.5 mL tube by mixing 50 µL (corresponding to 500 µg of extract) of the extract-containing liquid (10 mg/mL, containing 50% ethanol extract) prepared in Preparation Example 1 with 50 µL of pyridine. The solution in the tube was subjected to reduced pressure centrifugation treatment to remove the solvent, and an extract-containing powder was prepared. Then, 500 µL of pure water was added to the tube and mixed to prepare a mixed solution. The mixed solution was filtered through a 0.45 µm filter to remove solids, and a liquid composition C1 was obtained.

### (Comparative Example 2)

A liquid composition C2 was obtained in the same manner as in Comparative Example 1, except that DMSO was added instead of pure water.

### (Comparative Example 3)

A solution was prepared in a 1.5 mL tube by mixing 50 µL of the ellagic acid-containing solution (1 mg/mL) (corresponding to 50 µg of ellagic acid) prepared in Preparation Example 4 with 50 µL of a 50% aqueous ethanol solution. The solution in the tube was subjected to reduced pressure centrifugation treatment to remove the solvent, and an ellagic acid-containing powder was prepared. Then, 500 µL of hydrochloric acid (2 N) was added to the tube and mixed with the ellagic acid-containing powder to prepare a mixed solution. Then, the tube containing the mixed solution was placed in a block incubator and incubated at 95°C for 4 hours. Then, the mixed solution after incubation was left in the ambient environment to return to room temperature (23°C). Thereafter, the mixed solution was filtered through a 0.45 µm filter to remove solids, and a liquid composition C3 was obtained.

### (Comparative Example 4)

A solution was prepared in a 1.5 mL tube by mixing 50 µL of the ellagic acid-containing liquid (1 mg/mL) (corresponding to 50 µg of ellagic acid) prepared in Preparation Example 4 with 50 µL of a 50% aqueous ethanol solution. The solution in the tube was subjected to reduced pressure centrifugation treatment to remove the solvent, and an ellagic acid-containing powder was prepared. Then, 500 µL of pure water was added to the tube and mixed with the ellagic acid-containing powder to prepare a mixed solution. The mixed solution was filtered through a 0.45 µm filter to remove solids, and a liquid composition C4 was obtained.

### (Comparative Example 5)

A liquid composition C5 was obtained in the same manner as in Comparative Example 4, except that DMSO was added instead of pure water.

### (Evaluation)

The ellagic acid concentration of the obtained liquid composition was measured according to the above described measurement method. The results are shown in Table 2.

**[Table 2]**

| | Liquid composition | Material | Solvent | Heat treatment | Ellagic acid concentration µg/mL |
|---|---|---|---|---|---|
| Example 1 | E1 | Extract-containing solution | 2N Hydrochloric acid | 95°C for 4 hours | 114.5 |
| Comparative Example 1 | C1 | Extract-containing solution | Water | - | 27.6 |
| Comparative Example 2 | C2 | Extract-containing solution | DMSO | - | 27.7 |
| Comparative Example 3 | C3 | Ellagic acid-containing solution | 2N Hydrochloric acid | 95°C for 4 hours | 18.9 |
| Comparative Example 4 | C4 | Ellagic acid-containing solution | Water | - | 24.4 |
| Comparative Example 5 | C5 | Ellagic acid-containing solution | DMSO | - | 115.9 |

### (Discussion)

The liquid composition E1 obtained in Example 1 was obtained by subjecting a mixed solution prepared by adding hydrochloric acid to an extract derived from a plant of the genus Terminalia and subjecting the mixed solution to hydrolysis treatment by heating. The concentration of ellagic acid in the solution obtained after the hydrolysis treatment was 114.5 µg/mL, and the ellagic acid was dissolved in the solution at a high concentration. The solubility of ellagic acid in water is about 20 µg/mL, and thus in Example 1, the ellagic acid is solubilized to a level far exceeding the solubility. In Example 1, the extract-containing liquid (10 mg/mL) is mixed with pyridine and then the solvent is removed by a reduced pressure centrifugation treatment, but the mixing operation with pyridine was performed in connection with the experimental example described below from the viewpoints of conditions and the like, and is an operation that may be omitted in the present embodiment.

Discussion of Comparative Example 1 is as follows. The ellagic acid concentration in the liquid composition E1 of Example 1 was 114.5 µg/mL, whereas the ellagic acid concentration in the liquid composition C1 of Comparative Example 1 was 27.6 µg/mL. This is considered to be that in Example 1, the extract derived from a plant of the genus Terminalia was subjected to hydrolysis treatment by heating in the presence of hydrochloric acid, resulting in hydrolysis of the ellagitannins contained in the extract to produce ellagic acid, and the produced ellagic acid was solubilized to a concentration exceeding the saturation concentration in water by the action of the component contained in the extract derived from *Terminalia catappa.*

Discussion of Comparative Example 2 is as follows. DMSO, which has excellent solubility for ellagic acid, is used as the solvent in Comparative Example 2, and thus it is considered that the ellagic acid concentration in the liquid composition C2 of Comparative Example 2 indicates the amount of ellagic acid originally contained in the extract. The ellagic acid concentration in the liquid composition E1 of Example 1 was 114.5 µg/mL, and the ellagic acid concentration in the liquid composition C2 of Comparative Example 2, which indicates the amount of ellagic acid originally contained in the extract, was 27.7 µg/mL. This is considered to be that in Example 1, ellagitannin contained in the extract was hydrolyzed to produce ellagic acid.

The liquid composition C3 of Comparative Example 3 was obtained by adding hydrochloric acid to an ellagic acid-containing liquid (without an extract derived from a plant of the genus Terminalia) followed by heating, but this shows that a high-concentration ellagic acid-containing composition cannot be obtained by simply heating in hydrochloric acid without an extract derived from a plant of the genus Terminalia. Comparative Examples 4 and 5 are shown as controls in a case where pure water or DMSO is used, but Example 1 achieves an ellagic acid concentration as high as that of the liquid composition C5 of Comparative Example 5, which uses DMSO.

### [Investigation on improving ellagic acid solubility 1]

### (Example A1)

A solution was prepared in a 1.5 mL tube by mixing 50 µL (corresponding to 50 µg of ellagic acid) of the ellagic acid-containing solution (1 mg/mL) prepared in Preparation Example 4 and 50 µL (corresponding to 500 µg of extract) of the extract-containing solution (10 mg/mL, containing 50% ethanol extract) prepared in Preparation Example 1. The solution in the tube was subjected to reduced pressure centrifugation treatment to remove the solvent, and an ellagic acid + extract-containing powder was prepared. Then, 500 µL of pure water was added to the tube and mixed with the ellagic acid + extract-containing powder to prepare a mixed solution. Then, the tube containing the mixed solution was placed in a block incubator and incubated at 95°C for 20 minutes. Then, the mixed solution after incubation was left in the ambient environment to return to room temperature (23°C). Thereafter, the mixed solution was filtered through a 0.45 µm filter to remove solids, thereby obtaining a liquid composition E-A1.

### (Example A2)

A liquid composition E-A2 was obtained in the same manner as in Example A1, except that the heating temperature in the block incubator was changed to 75°C.

### (Example A3)

A liquid composition E-A3 was obtained in the same manner as in Example A1, except that heating was performed at 121°C using an autoclave instead of heating in a block incubator.

### (Example A4)

A liquid composition E-A4 was obtained in the same manner as in Example A1, except that the heating temperature in the block incubator was changed to 55°C.

### (Comparative Example A1)

Liquid composition C-A1 was obtained in the same manner as in Example A1, except that the mixture was left at room temperature (23°C) for 20 minutes without being heated in a block incubator.

### (Example B1)

A solution was prepared in a 1.5 mL tube by mixing 50 µL of pyridine (no ellagic acid added) and 50 µL (corresponding to 500 µg of extract) of the extract-containing liquid (10 mg/mL, containing 50% ethanol extract) prepared in Preparation Example 1. The solution in the tube was subjected to reduced pressure centrifugation treatment to remove the solvent, and an extract-containing powder was prepared. Then, 500 µL of pure water was added to the tube and mixed with the extract-containing powder to prepare a mixed solution. Then, the tube containing the mixed solution was placed in a block incubator and incubated at 95°C for 20 minutes. Then, the mixed solution after incubation was left in the ambient environment to return to room temperature (23°C). Thereafter, the mixed solution was filtered through a 0.45 µm filter to remove solids, and a liquid composition E-B1 was obtained.

### (Example B2)

A liquid composition E-B2 was obtained in the same manner as in Example B1, except that the heating temperature in the block incubator was changed to 75°C.

### (Example B3)

A liquid composition E-B3 was obtained in the same manner as in Example B1, except that heating was performed at 121°C using an autoclave instead of heating in a block incubator.

### (Example B4)

A liquid composition E-B4 was obtained in the same manner as in Example B1, except that the heating temperature in the block incubator was changed to 55°C

### (Comparative Example B1)

A liquid composition C-B1 was obtained in the same manner as in Example B1, except that the mixture was left at room temperature (23°C) for 20 minutes without being heated in a block incubator.

### (Comparative Example B2)

A solution was prepared in a 1.5 mL tube by mixing 50 µL of pyridine (no ellagic acid added) and 50 µL (corresponding to 500 µg of extract) of the extract-containing liquid (10 mg/mL, containing 50% ethanol extract) prepared in Preparation Example 1. The solution in the tube was subjected to reduced pressure centrifugation treatment to remove the solvent, and an extract-containing powder was prepared. Then, 500 µL of DMSO was added to the tube and mixed with the extract-containing powder to prepare a mixed solution. Thereafter, the mixed solution was filtered through a 0.45 µm filter to remove solids, and a liquid composition C-B2 was obtained.

### (Comparative Example C1)

A solution was prepared in a 1.5 mL tube by mixing 50 µL of the ellagic acid-containing solution (1 mg/mL) (corresponding to 50 µg of ellagic acid) prepared in Preparation Example 4 with 50 µL of 50% ethanol aqueous solution (no extract added). The solution in the tube was subjected to reduced pressure centrifugation treatment to remove the solvent, and an ellagic acid-containing powder was prepared. Then, 500 µL of pure water was added to the tube and mixed with the ellagic acid-containing powder to prepare a mixed solution. Then, the tube containing the mixed solution was placed in a block incubator and incubated at 95°C for 20 minutes. Then, the mixed solution after incubation was left in the ambient environment to return to room temperature (23°C). Thereafter, the mixed solution was filtered through a 0.45 µm filter to remove solids, and a liquid composition C-C1 was obtained.

### (Comparative Example C2)

A liquid composition C-C2 was obtained in the same manner as in Comparative Example C1, except that the heating temperature in the block incubator was changed to 75°C.

### (Comparative Example C3)

A liquid composition C-C3 was obtained in the same manner as in Comparative Example C1, except that heating was performed at 121°C using an autoclave instead of heating in a block incubator.

### (Comparative Example C4)

A liquid composition C-C4 was obtained in the same manner as in Comparative Example C1, except that the heating temperature in the block incubator was changed to 55°C.

### (Comparative Example C5)

A liquid composition C-C5 was obtained in the same manner as in Comparative Example C1, except that the mixture was left at room temperature (23°C) for 20 minutes without being heated in a block incubator.

### (Comparative Example C6)

A solution was prepared in a 1.5 mL tube by mixing 50 µL of the ellagic acid-containing liquid (1 mg/mL) (corresponding to 50 µg of ellagic acid) prepared in Preparation Example 4 with 50 µL of a 50% aqueous ethanol solution (no extract added). The solution in the tube was subjected to reduced pressure centrifugation treatment to remove the solvent, and an ellagic acid-containing powder was prepared. Then, 500 µL of DMSO was added to the tube and mixed with the ellagic acid-containing powder to prepare a mixed solution. Then, the mixed solution was filtered through a 0.45 µm filter to remove solids, and a liquid composition C-C6 was obtained.

### (Comparative Example D1)

A solution was prepared in a 1.5 mL tube by mixing 50 µL (corresponding to 50 µg of ellagic acid) of the ellagic acid-containing solution (1 mg/mL) prepared in Preparation Example 4 and 50 µL (corresponding to 500 µg of extract) of the extract-containing solution (10 mg/mL, containing 50% ethanol extract) prepared in Preparation Example 1. The solution in the tube was subjected to reduced pressure centrifugation treatment to remove the solvent, and an ellagic acid + extract-containing powder was prepared. Then, 500 µL of DMSO was added to the tube and mixed with the ellagic acid + extract-containing powder to prepare a mixed solution. Thereafter, the mixed solution was filtered through a 0.45 µm filter to remove solids, and a liquid composition C-D1 was obtained.

### (Evaluation)

### <Measurement of ellagic acid concentration>

The ellagic acid concentration of the obtained liquid composition was measured according to the above described measurement method. The results are shown in Table 3.

**[Table 3]**

| | Liquid composition | Ellagic acid added µg | Extract added µg | Solvent | Heating temperature °C | Heating time minutes | Ellagic acid concentration µg/mL |
|---|---|---|---|---|---|---|---|
| Example A1 | E-A1 | 50 | 500 | Water | 95 | 20 | 106.0 |
| Example A2 | E-A2 | 50 | 500 | Water | 75 | 20 | 71.2 |
| Example A3 | E-A3 | 50 | 500 | Water | 121 | 20 | 124.0 |
| Example A4 | E-A4 | 50 | 500 | Water | 55 | 20 | 33.4 |
| Comparative Example A1 | C-A1 | 50 | 500 | Water | Room temperature | 20 | 27.2 |
| Example B1 | E-B1 | - | 500 | Water | 95 | 20 | 242 |
| Example B2 | E-B2 | - | 500 | Water | 75 | 20 | 23.0 |
| Example B3 | E-B3 | - | 500 | Water | 121 | 20 | 37.2 |
| Example B4 | E-B4 | - | 500 | Water | 55 | 20 | 22.1 |
| Comparative Example B1 | C-B1 | - | 500 | Water | Room temperature | 20 | 20.8 |
| Comparative Example B2 | C-B2 | - | 500 | DMSO | - | - | 21.2 |
| Comparative Example C1 | C-C1 | 50 | - | Water | 95 | 20 | 15.4 |
| Comparative Example C2 | C-C2 | 50 | - | Water | 75 | 20 | 15.4 |
| Comparative Example C3 | C-C3 | 50 | - | Water | 121 | 20 | 27.3 |
| Comparative Example C4 | C-C4 | 50 | - | Water | 55 | 20 | 15.6 |
| Comparative Example C5 | C-C5 | 50 | - | Water | Room temperature | 20 | 20.4 |
| Comparative Example C6 | C-C6 | 50 | - | DMSO | - | - | 88.2 |
| Comparative Example D1 | C-D1 | 50 | 500 | DMSO | - | - | 101.5 |

### <Calculation of solubilization rate>

The solubilization rate of the added ellagic acid in Examples A1 to A4 and Comparative Example A1 was calculated using the above results. The solubilization rate was calculated based on the solubilization degree in a case of using DMSO as a solvent. The solubilization degree in a case of using DMSO as a solvent was a value (80.3 µg/mL) obtained by subtracting the ellagic acid concentration of the liquid composition C-B2 of Comparative Example B2 (21.2 µg/mL) from the liquid composition C-D1 of Comparative Example D1 (101.5 µg/mL). Specifically, the solubilization degree of solubilized portion of the added ellagic acid was calculated by subtracting the ellagic acid concentration obtained in the corresponding example from the ellagic acid concentration obtained in Example or Comparative Example, and the solubilization rate (%) was calculated by dividing it by the above 80.3 µg/mL and multiplying by 100. Example A1 corresponds to Example B1, Example A2 corresponds to Example B2, Example A3 corresponds to Example B3, Example A4 corresponds to Example B4, and Comparative Example A1 corresponds to Comparative Example B1. The calculated solubilization rates are shown in Table 4.

**[Table 4]**

| | Solubilization rate % |
|---|---|
| Example A1 | 101.8 |
| Example A2 | 60.1 |
| Example A3 | 108.1 |
| Example A4 | 14.1 |
| Comparative Example A1 | 8.0 |

### (Discussion)

As shown in Examples A1 to A4, it was confirmed that adding ellagic acid to an extract derived from a plant of the genus Terminalia followed by heating can dissolve the added ellagic acid at a high concentration. This is presumably because the extract derived from a plant of the genus Terminalia contains a component that solubilizes ellagic acid, and heat treatment in the presence of this component can solubilize ellagic acid.

### [Investigation on improving ellagic acid solubility 2]

### (Example A5)

A liquid composition E-A5 was obtained in the same manner as in Example A1, except that the extract-containing liquid (10 mg/mL, containing water extract) prepared in Preparation Example 2 was used.

### (Example A6)

A liquid composition E-A6 was obtained in the same manner as in Example A5, except that heating was performed at 121°C using an autoclave instead of heating in a block incubator.

### (Example A7)

A liquid composition E-A7 was obtained in the same manner as in Example A1, except that the extract-containing liquid (10 mg/mL, containing 100% ethanol extract) prepared in Preparation Example 3 was used.

### (Example A8)

A liquid composition E-A8 was obtained in the same manner as in Example A7, except that heating was performed at 121°C using an autoclave instead of heating in a block incubator.

### (Example B5)

A liquid composition E-B5 was obtained in the same manner as in Example B1, except that the extract-containing liquid (10 mg/mL, containing water extract) prepared in Preparation Example 2 was used.

### (Example B6)

A liquid composition E-B6 was obtained in the same manner as in Example B5, except that heating was performed at 121°C using an autoclave instead of heating in a block incubator.

### (Example B7)

A liquid composition E-B7 was obtained in the same manner as in Example B1, except that the extract-containing liquid (10 mg/mL, containing 100% ethanol extract) prepared in Preparation Example 3 was used.

### (Example B8)

A liquid composition E-B8 was obtained in the same manner as in Example B7, except that heating was performed at 121°C using an autoclave instead of heating in a block incubator.

### (Comparative Example E1)

A liquid composition C-E1 was obtained in the same manner as in Example A5, except that the mixture was left at room temperature (23°C) for 20 minutes without being heated in a block incubator.

### (Comparative Example E2)

A liquid composition C-E2 was obtained in the same manner as in Example A7, except that the mixture was left at room temperature (23°C) for 20 minutes without being heated in a block incubator.

### (Comparative Example E3)

A liquid composition C-E3 was obtained in the same manner as in Example B5, except that the mixture was left at room temperature (23°C) for 20 minutes without being heated in a block incubator.

### (Comparative Example E4)

A liquid composition C-E4 was obtained in the same manner as in Example B7, except that the mixture was left at room temperature (23°C) for 20 minutes without being heated in a block incubator.

### (Comparative Example F1)

A liquid composition C-F1 was obtained in the same manner as in Comparative Example D1, except that the extract-containing liquid (10 mg/mL, containing water extract) prepared in Preparation Example 2 was used.

### (Comparative Example F2)

A liquid composition C-F2 was obtained in the same manner as in Comparative Example D1, except that the extract-containing liquid (10 mg/mL, containing 100% ethanol extract) prepared in Preparation Example 3 was used.

### (Comparative Example F3)

A liquid composition C-F3 was obtained in the same manner as in Comparative Example B2, except that the extract-containing liquid (10 mg/mL, containing water extract) prepared in Preparation Example 2 was used.

### (Comparative Example F4)

A liquid composition C-F4 was obtained in the same manner as in Comparative Example B2, except that the extract-containing liquid (10 mg/mL, containing 100% ethanol extract) prepared in Preparation Example 3 was used.

### (Evaluation)

### <Measurement of ellagic acid concentration>

The ellagic acid concentration of the obtained liquid composition was measured according to the above described measurement method. The results are shown in Table 5.

**[Table 5]**

| | Liquid composition | Ellagic acid added µg | Extract added µg | Extraction solvent | Solvent | Heating temperature °C | Heating time minutes | Ellagic acid concentration µg/mL |
|---|---|---|---|---|---|---|---|---|
| Example A5 | E-A5 | 50 | 500 | Water | Water | 95 | 20 | 102.8 |
| Example A6 | E-A6 | 50 | 500 | Water | Water | 121 | 20 | 107.5 |
| Example A7 | E-A7 | 50 | 500 | 100%EtOH | Water | 95 | 20 | 82.5 |
| Example A8 | E-A8 | 50 | 500 | 100%EtOH | Water | 121 | 20 | 118.3 |
| Example B5 | E-B5 | - | 500 | Water | Water | 95 | 20 | 19.2 |
| Example B6 | E-B6 | - | 500 | Water | Water | 121 | 20 | 29.5 |
| Example B7 | E-B7 | - | 500 | 100%EtOH | Water | 95 | 20 | 243 |
| Example B8 | E-B8 | - | 500 | 100%EtOH | Water | 121 | 20 | 35.6 |
| Comparative Example E1 | C-E1 | 50 | 500 | Water | Water | Room temperature | 20 | 31.1 |
| Comparative Example E2 | C-E2 | 50 | 500 | 100%EtOH | Water | Room temperature | 20 | 22.0 |
| Comparative Example E3 | C-E3 | - | 500 | Water | Water | Room temperature | 20 | 17.5 |
| Comparative Example E4 | C-E4 | - | 500 | 100%EtOH | Water | Room temperature | 20 | 21.4 |
| Comparative Example F1 | C-F1 | 50 | 500 | Water | DMSO | - | - | 105.7 |
| Comparative Example F2 | C-F2 | 50 | 500 | 100%EtOH | DMSO | - | - | 123.5 |
| Comparative Example F3 | C-F3 | - | 500 | Water | DMSO | - | - | 18.4 |
| Comparative Example F4 | C-F4 | - | 500 | 100%EtOH | DMSO | - | - | 22.0 |

### <Calculation of solubilization rate>

Using the above results, the solubilization rates of the added ellagic acid in Examples A5 to A8 and Comparative Examples E1 to E2 were calculated in the same manner as described above. Example A5 corresponds to Example B5, Example A6 corresponds to Example B6, Example A7 corresponds to Example B7, Example A8 corresponds to Example B8, Comparative Example E1 corresponds to Comparative Example E3, and Comparative Example E2 corresponds to Comparative Example E4. The solubilization degree in a case of using DMSO, which was used as the standard for calculating the solubilization rates of Examples A5, A6, and Comparative Example E1, as the solvent was a value (87.3 µg/mL) obtained by subtracting the ellagic acid concentration of Comparative Example F3 (18.4 µg/mL) from the ellagic acid concentration of Comparative Example F1 (105.7 µg/mL), and the solubilization degree in a case of using DMSO, which was used as the standard for calculating the solubilization rates of Examples A7, A8, and Comparative Example E2, as the solvent was a value (101.5 µg/mL) obtained by subtracting the ellagic acid concentration of Comparative Example F4 (22.0 µg/mL) from the ellagic acid concentration of Comparative Example F2 (123.5 µg/mL). The calculated solubilization rates are shown in Table 6.

**[Table 6]**

| | Solubilization rate % |
|---|---|
| Example A5 | 95.8 |
| Example A6 | 89.3 |
| Example A7 | 57.3 |
| Example A8 | 81.6 |
| Comparative Example E1 | 15.5 |
| Comparative Example E2 | 0.7 |

### (Discussion)

As shown in Examples A5 to A8, the solubilization rate of ellagic acid was improved even in a case of using extracts with water and 100% ethanol. As a result, the solubilization rate of ellagic acid was higher in the extract with water than in the extract with 100% ethanol.

### [Investigation on improving ellagic acid solubility 3]

### (Example A9)

A solution was prepared in a 1.5 mL tube by mixing 50 µL of the ellagic acid-containing solution (1 mg/mL) (corresponding to 50 µg of ellagic acid) prepared in Preparation Example 4 and 50 µL of the extract-containing solution (10 mg/mL, containing 50% ethanol extract) (corresponding to 500 µg of extract) prepared in Preparation Example 1. The solution in the tube was subjected to reduced pressure centrifugation treatment to remove the solvent, and an extract-containing powder was prepared. Then, 500 µL of pure water was added to the tube and mixed with the extract-containing powder to prepare a mixed solution. Then, the tube containing the mixed solution was placed in a block incubator and incubated at 95°C for 20 minutes. Then, the mixed solution after incubation was left in the ambient environment to return to room temperature (23°C). Then, the mixed solution in the tube was concentrated by reduced pressure centrifugation until the volume was about 1/10. Thereafter, the mixed solution was filtered through a 0.45 µm filter to remove solids, thereby providing a liquid composition E-A9.

### (Example A10)

A liquid composition E-A10 was obtained in the same manner as in Example A9, except that the extract-containing liquid prepared in Preparation Example 2 (10 mg/mL, containing water extract) was used instead of the extract-containing liquid prepared in Preparation Example 1 (10 mg/mL, containing 50% ethanol extract).

### (Evaluation)

### <Measurement of ellagic acid concentration>

The ellagic acid concentration of the obtained liquid composition was measured according to the above described measurement method. The results are shown in Table 7.

**[Table 7]**

| | Liquid composition | Ellagic acid concentration µg/mL |
|---|---|---|
| Example A9 | E-A9 | 942.7 |
| Example A10 | E-A10 | 833.5 |

### (Discussion)

As shown in Examples A9 to A10, it was confirmed that an ellagic acid-containing composition with a higher concentration can be obtained by further concentrating the heat-treated composition containing an extract derived from a plant of the genus Terminalia. Considering that the solubility of ellagic acid in water is about 20 µg/mL, it can be understood that the solubilization effect of ellagic acid obtained by heat treatment of an extract derived from a plant of the genus Terminalia is extremely remarkable.

The upper and/or lower limit values of the numerical ranges described in the present description can be combined as desired to define a preferable range. For example, the upper and lower limit values of the numerical ranges can be combined as desired to define a preferable range, the upper limit values of the numerical ranges can be combined as desired to define a preferable range, and the lower limit values of the numerical ranges can be combined as desired to define a preferable range.

The claims that follow this written disclosure are expressly incorporated herein into this written disclosure, with each claim standing on its own as a separate embodiment. The present disclosure includes any substitution of an independent claim with its dependent claim. Further, any additional embodiments that derive from the independent claims and the subsequent dependent claims are also expressly incorporated into this written description.

Those skilled in the art can use the above description to make the most of the present disclosure. The claims and embodiments disclosed herein are merely descriptive and exemplary and should not be construed as limiting the scope of the present disclosure in any way. With the aid of the present disclosure, changes can be made in the details of the above embodiments without departing from the basic principles of the present disclosure. In other words, various modifications and improvements of the embodiments specifically disclosed in the above description are within the scope of the present disclosure.

Although the present embodiment has been described in detail above, the specific configuration is not limited to the present embodiment, and even if there are design changes that do not deviate from the gist of the present disclosure, they are included in the present disclosure.

## Claims

1. A method for producing an ellagic acid-containing composition, comprising:
a step of providing a solution containing at least an aqueous medium and a raw material derived from a plant of the genus *Terminalia,* containing ellagic acid; and
a step of heat-treating the solution.

2. The production method according to claim 1, further comprising a step of a hydrolysis treatment to hydrolyze ellagitannin contained in the raw material to produce additional ellagic acid, wherein the hydrolysis-treated solution is heat-treated.

3. The production method according to claim 2, wherein the hydrolysis treatment is hydrolysis with an acid.

4. The production method according to any one of claims 1 to 3, wherein the solution further contains ellagic acid added separately from ellagic acid contained in a raw material derived from a plant of the genus *Terminalia.*

5. The production method according to any one of claims 1 to 4, wherein the plant of the genus *Terminalia* is *Terminalia catappa.*

6. The production method according to any one of claims 1 to 5, wherein the raw material derived from a plant of the genus *Terminalia* is at least one material selected from leaves, fruits, stems, and branches of a plant of the genus *Terminalia,* or is an extract extracted from at least one material selected from leaves, fruits, stems, and branches of a plant of the genus *Terminalia* with an extraction medium.

7. The production method according to any one of claims 1 to 6, wherein the raw material derived from a plant of the genus *Terminalia* is an extract extracted from at least one material selected from leaves, fruits, stems, and branches of a plant of the genus *Terminalia* with an extraction medium.

8. The production method according to claim 6 or 7, wherein the extraction medium is water, an aqueous medium, or a mixed solvent thereof.

9. The production method according to any one of claims 6 to 8, wherein the extraction medium is water, an alcohol, or a mixed solvent of water and an alcohol.

10. The production method according to any one of claims 1 to 9, wherein a temperature for the heat treatment is 60°C or more.

11. The production method according to any one of claims 1 to 10, wherein a temperature for the heat treatment is less than 100°C.

12. The production method according to any one of claims 1 to 11, wherein the aqueous medium in the solution is water, alcohol, or a mixed solvent of water and alcohol.

13. The production method according to any one of claims 1 to 12, further comprising a step of concentration-treating a solution after the heat-treating step.

14. The production method according to any one of claims 1 to 13, wherein a concentration of ellagic acid dissolved in a solution obtained by the heat-treating step, or the concentration-treating step when performing the concentration treatment is 50 µg/mL or more.

15. The production method according to any one of claims 1 to 14, further comprising a step of dry-treating the solution, after the heat-treating step, or after the concentration-treating step when performing the concentration treatment, to obtain an ellagic acid-containing solid.

16. An ellagic acid-containing composition obtained by the production method according to any one of claims 1 to 15.

17. A medicine, cosmetic, quasi-drug, or food or drink comprising the ellagic acid-containing composition according to claim 16.

18. Use of a raw material derived from a plant of the genus *Terminalia* for solubilizing ellagic acid, wherein a solution containing a raw material derived from a plant of the genus *Terminalia* and an aqueous medium is heat-treated in the presence of ellagic acid.
